# EUROPEAN PATENT APPLICATION

(11) **EP 1 149 822 A1**
(43) Date of publication of application: **31.10.2001**
(21) Application number: 00201543.6
(22) Date of filing: 28.04.2000
(51) Int. Cl.: C07C 51/43, C07C 59/105

(54) **Ferrous salt of D-gluconic acid dihydrate substantially free of Ba2+ and SO4 2- ions**

(71) Applicant: AZIENDE CHIMICHE RIUNITE ANGELINI FRANCESCO A.C.R.A.F. S.p.A., 00181 Roma (IT)
(72) Inventor: Giannangeli, Marilena, 00181 Roma (IT)
(74) Representative: Marchi, Massimo, Dr.

(57) **Abstract**

Ferrous salt of D-gluconic acid dihydrate of formula (I) 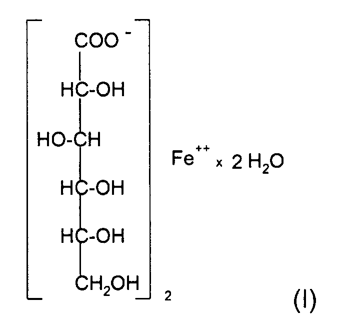 substantially free of Ba²⁺ and SO0₄²⁻ ions, and process for preparing the same.

## Description

The present invention relates to a ferrous salt of D-gluconic acid dihydrate which is substantially free of Ba²⁺ and SO₄²⁻ ions.

It is known that the ferrous salt of D-gluconic acid dihydrate of formula (I) 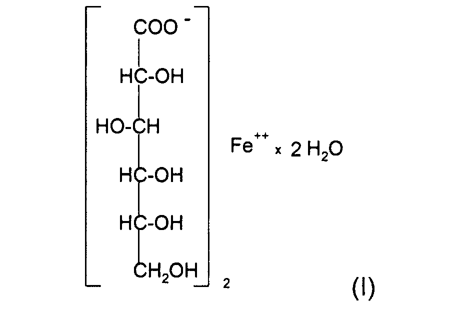 which is commonly sold contains Ba²⁺ and SO₄²⁻ ions since it is prepared by double exchange between ferrous sulphate and barium gluconate.

The attempts made hitherto to remove the abovementioned ions have not given satisfactory results.

Now, it has been surprisingly found that this can be achieved by dissolving the ferrous salt D-gluconic acid dihydrate of formula (I), containing Ba²⁺ and SO₄²⁻ ions, in water and then reprecipitating it by addition of at least four volumes of ethanol.

Therefore, it is a first object of the present invention to provide the ferrous salt of D-gluconic acid dihydrate of formula (I) 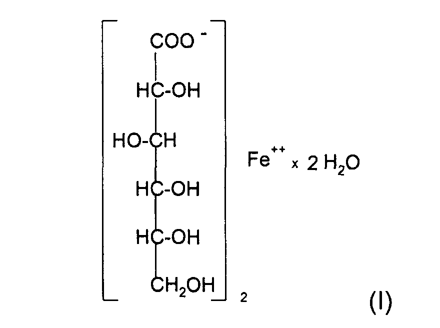 which is substantially free of Ba²⁺ and SO₄²⁻ ions.

Further, it is a second object of the present invention to provide a method for obtaining the ferrous salt of D-gluconic acid dihydrate of formula (I) 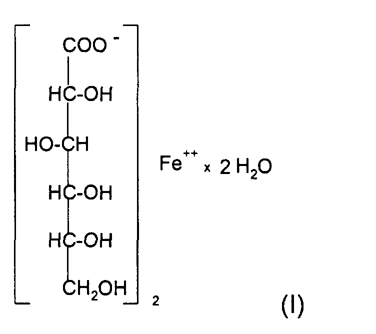 which is substantially free of Ba²⁺ and SO₄²⁻ ions, characterized in that it comprises the steps of
a) dissolving the said compound of formula (I), containing Ba²⁺ and SO₄²⁻ ions, in water, and
b) reprecipitating the said compound of formula (I) from ethanol by addition of at least four volumes of ethanol.

The example which follows is given to illustrate the present invention without, however, limiting it in any way.

### EXAMPLE

The ferrous salt of D-gluconic acid dihydrate (3 g), containing Ba²⁺ and SO₄²⁻ ions, was dissolved in water (80 ml) at room temperature and then reprecipitated by addition of ethanol (330 ml).

The ferrous salt of D-gluconic acid dihydrate having a Ba²⁺ and SO₄²⁻ ion content < 5 ppm, m.p. = 189°C (decomposition) and α^{D} = + 6.8 ( c = 1 H₂O) was thus obtained.

## Claims

1. Ferrous salt of D-gluconic acid dihydrate of formula (I) 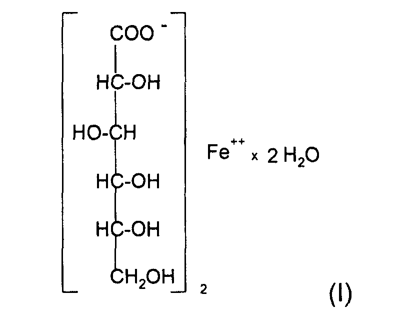 which is substantially free of Ba²⁺ and SO₄²⁻ ions.

2. Method for obtaining the ferrous salt of D-gluconic acid dihydrate of formula (I) 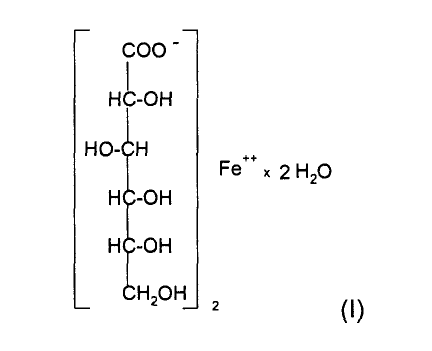 which is substantially free of Ba²⁺ and SO₄²⁻ ions, **characterized in that** it comprises the steps of
a) dissolving the said compound of formula (I), containing Ba²⁺ and SO₄²⁻ ions, in water, and
b) reprecipitating the said compound of formula (I) from ethanol by addition of at least four volumes of ethanol.
